# EUROPEAN PATENT APPLICATION

(11) **EP 1 504 754 A1**
(43) Date of publication of application: **09.02.2005**
(21) Application number: 03723329.3
(22) Date of filing: 12.05.2003
(51) Int. Cl.: A61K 7/26, A61K 31/353, A61K 35/78, A61P 1/02, A61P 31/04, A61P 43/00, C07D 311/60, A23L 1/03

(54) **GALLOCATECHIN GALLATE-CONTAINING COMPOSITION**

(30) Priority: 10.05.2002 JP 2002136081
(71) Applicant: SUNTORY LIMITED, Osaka-shi, Osaka 530-8203 (JP)
(72) Inventor: MAEDA, Mitsuru, Otsu-shi, Shiga 520-0244 (JP)
(74) Representative: VOSSIUS & PARTNER
(86) International application number: PCT/JP2003/005884
(87) International publication number: WO 2003/094878

(57) **Abstract**

The present invention provides a safe and highly effective antibacterial composition without impairing the flavor of foods or drinks, which composition is used in products such as foods and oral care goods which are mainly to be taken into human or animal bodies or mainly to be used in the oral cavity.

The composition according to the present invention contains gallocatechin gallate as an agent for enhancing the activity of antibacterial catechins in which the antibacterial activity of the antibacterial catechins against *Streptococcus mutans* is particularly enhanced. The composition according to the present invention can be produced by mixing the antibacterial catechins with gallocatechin gallate. Alternatively, it can be produced as a composition comprising a synthetic adsorbent-adsorbed fraction as the main component, which is obtained by subjecting a solvent-extract from tea leaves to an adsorption treatment with the use of a synthetic adsorbent selected from among aromatic compound-based synthetic adsorbents and methacrylic compound-based synthetic adsorbents.

## Description

### TECHNICAL FIELD

This invention relates to a composition comprising an agent having an antibacterial activity, such as (-)-epigallocatechin gallate (EGCG) mixed with its epimer gallocatechin gallate (GCG), whereby the antibacterial activity of EGCG is enhanced, as well as foods and drinks containing the composition.

### BACKGROUND ART

With the recent growing interest in health, attempts have been made to reduce the contents of salt and sugar in foods and drinks. As a result, a problem arises that humidity is elevated in foods, which facilitates the proliferation of microorganisms. It is reported that about 87% or more cases of food poisoning are caused by bacteria (Yoji Kato, Aug. 2001, *Gekkan Food Chemical,* published by Shokuhin Kagaku Shinbun-sha Inc.). Although refrigerated distribution systems have been developed in these days, it is still urgently required to ensure storage stability and safety of foods. For this purpose, it has been a common practice to use food additives, for example, preservatives such as sorbic acid (or potassium salt thereof), benzoic acid (or sodium salt thereof) and polylysine, and storage stability-improvers such as glycerine fatty acid esters, glycine and tea extract. Use of chemically synthesized preservatives, which are not always safe with regard to toxicity, skin irritation and allergy, is strictly regulated. Most of the existing storage stability-improvers comprise essential oil components as active ingredients, but these components can be used only in a limited scope or in a limited amount because of the distinctive, strong smell and low solubility in water. Further, tea extract has another problem that the bitterness and astringency of catechins contained therein become perceivable at a concentration where the tea extract can exhibit the antibacterial effect. Although there have been disclosed methods of using saccharides (for example, a method disclosed in JP-A-H8-298930 wherein dextrin and an enzyme reaction are used, and a method disclosed in JP-A-H3-168046 wherein cyclodextrin is concomitantly used), and a method of using proteins (for example, a method disclosed in JP-A-H2-202900 wherein egg albumin, vegetable protein and so on are used, and a method disclosed in JP-A-2001-31669 wherein defatted egg yolk is concomitantly used), it is still required to develop a food additive capable of exerting an antibacterial activity at a lower concentration and being not restricted in terms of application scope or amount, thereby satisfying customers' demands for safety and reliability.

From the viewpoint of oral hygiene, on the other hand, oral bacteria cause serious problems of dental caries and periodontal disease. With regard to the pathogen of dental caries, it is recognized that dental caries falls within the category of bacterial infections based on the chemico-parasitic theory. According to this theory, the onset mechanism of dental caries is as follows. First, enzyme glucosyl transferase produced by oral streptococci, in particular, *Streptococcus mutans,* generates sticky, insoluble polysaccharides (glucans), utilizing sucrose in the mouth as the substrate. Due to the glucans thus formed, cells of the oral streptococci adhere to tooth surface to form massed bacterias (dental plaque). In the dental plaque, various microorganisms live and proliferate and, as a result, produce organic acids as their metabolites. These organic acids lower the pH value on the tooth surface, and the enamel layer on the surface is decalcified. Dental caries thus occurs and proceeds. Further, it is reported that the plaque formation causes not only dental caries but also periodontal diseases and oral odor (Shigeyuki Hamada, Feb. 1982, Iwanami Shinsho, *Mushiba wa Dohshite Dekiruka,* published by Iwanami Shoten). A large number of attempts have been made to prevent dental caries and periodontal disease. That is, research and development has been carried out on antibacterial agents for inhibiting the proliferation of oral microorganisms, for example, green tea catechins; inhibitors for glucosyl transferase produced by oral microorganisms, for example, polyphenols from oolong tea; and noncariogenic sugars unusable as a substrate in plaque formation, for example, xylitol. In recent years, plant components, in particular, polyphenols, have attracted public attention as cariostatic agents. For example, leaves of tea plant (*Camellia sinensis*) contain polyphenols in an amount as much as 36% on a dry weight basis, and the major components of the polyphenols are called green tea catechins. Catechins include (-)-epicatechin (EC), (-)-epigallocatechin (EGC), (-)-epicatechin gallate (ECG) and (-)-epigallocatechin gallate (EGCG). Among all, it is known that EGCG exerts an antibacterial effect, an antimutagenic effect and a favorable effect on blood cholesterol level. JP-A-2001-97968 reports a method of producing EGCG. Further, (+)-catechin (C), EC, (+)-gallocatechin (GC), EGC, ECG and EGCG are described as active ingredients of anti periodontal disease compositions in JP-A-H9-110687.

Alternatively, JP-A-H9-132532 describes a method of enhancing the antimicrobial activity of an antibiotic agent against methicillin-resistant *Staphylococcus aureus* (MRSA) by adding tea catechins and theaflavins. According to this report, EGCG and ECG can enhance the antibacterial activity of an antibiotic agent against MRSA.

To provide a highly safe and less expensive antibiotic agent originating in natural products, JP-A-H11-116418 discloses a method of producing a catechin-origin antimicrobial agent from green tea by the specified extraction conditions in terms of heating temperature, time and the like.

Furthermore, JP-A-2001-97968 as cited above discloses a method of purifying EGCG from a green tea extract, as well as an extract and a concentrate containing EGCG and GCG. However, an excellent anti *Streptococcus mutans* effect achieved by compounding EGCG and GCG is neither disclosed nor suggested in JP-A-2001-97968.

As discussed above, there has been found no antibacterial composition for preventing dental caries which is safe, inexpensive and satisfactory, and can be used without impairing the flavor of foods or drinks.

### SUMMARY OF THE INVENTION

The present invention provides an antibacterial composition for safely and effectively reducing or preventing the onset of dental caries without impairing the flavor of foods or drinks, which composition is used in products such as foods and oral care goods which are mainly to be taken into human or animal bodies, or mainly to be used in the oral cavity.

### DETAILED DESCRIPTION OF THE INVENTION

To search for a highly safe antibacterial agent with the use of the effect on *Streptococcus mutans* as an indication, the present inventors have conducted intensive studies and, as a result, surprisingly found that GCG (gallocatechin gallate) has an activity of enhancing the antibacterial activity of green tea catechins such as EGCG, thereby completing the present invention.

Accordingly, the present invention relates to a composition comprising catechins mixed with an antibacterial effect enhancer gallocatechin gallate.

The present invention also relates to an antibacterial composition against *Streptococcus mutans* and the like, containing an antibacterial-active fraction containing gallocatechin gallate and green tea catechins obtained by extracting tea leaves with a solvent, adsorbing the solvent-extract from the tea leaves on synthetic adsorbent selected from among aromatic compound-based synthetic adsorbents and methacrylic compound-based synthetic adsorbents and then eluting the adsorbed components from the adsorbent, where the antibacterial activity of the green tea catechins is enhanced by the coexisting gallocatechin gallate.

The term "green tea catechins" as used in the present invention means green tea polyphenol catechins known as a composition having very safe cariostatic and anti periodontal disease activity based on the antibacterial activity, and includes for example, C, EC, GC, EGC, ECG, EGCG, etc., or a mixture thereof. It is preferable that the green tea catechins contain at least EGCG.

The term "antibacterial catechins" as used in the present invention means catechins at least containing EGCG and having an antibacterial effect.

The composition according to the present invention is an antibacterial composition that is expected to be applicable in the fields of quasi drugs or foods, that exerts a highly safe, antibacterial, cariostatic, and anti periodontal disease effects, and that is aimed at improving the storage stability of drinks or processed foods.

Although the composition according to the present invention may be produced from any starting materials without restriction, preferably it derives from tea in view of stable supply. The "tea" as used herein includes, but is not limited to, unfermented tea products of green tea such as *Sen-cha* (Japanese green tea), *Houji-cha* (roasted green tea), *gyokuro tea, Kabuse-cha* (semi sun shaded tea), and *Mushi-seicha* (steamed tea); unfermented tea products of various Chinese type green tea (*Kamairi-cha,* pan fired tea), such as *Ureshino-cha, Aoyagi-cha;* semi-fermented tea products such as *Hoshu-tea* and oolong tea; fermented tea products such as black tea, *Awa-bancha, Goishi-cha* and Pu-er tea; Mate-tea and so on. Considering the presence of an epimer and the synergistic effect on catechins, oolong tea produced from *Camellia* leaves is preferably employed as a supply source.

The composition according to the present invention may be produced by mixing GCG with catechins having an antibacterial effect, such as EGCG. Alternatively, it can be obtained using any of the tea materials described above as a starting material, according to a conventional extraction method. An extract from oolong tea using hot water or water-containing ethanol, etc., or an eluate which is obtained, as will be described in greater detail hereinafter, by dissolving or suspending said extract in water and then subjecting it to column chromatography using adsorptive resin such as DIAION HP-21 (manufactured by Mitsubishi Chemical Corporation) may be used.

The solvent used for extraction may be water alone, or an arbitrary mixture of water with one or more of polar solvents such as lower alcohols including methanol, ethanol, etc., and acetone. However, since the active ingredients according to the present invention cannot be efficiently extracted with a polar solvent alone, it is preferable to use a mixture of a polar solvent with water. Further, in such a solvent mixture, the content of the polar solvent is preferably not more than 90% by volume. Among these solvents, it is preferable to use water, ethanol or a mixture thereof from the viewpoint of safety, since the extract is to be finally formulated in oral care products or foods.

In the extraction step, the ratio of the tea leaves to the solvent is not particularly restricted. However, it is preferable to employ the solvent in an amount of 2 to 1,000 times by weight of the tea leaves, and in view of extraction procedure and efficiency, still preferably 5 to 100 times by weight of the tea leaves. It is convenient to carry out the extraction at a temperature of from room temperature to the boiling point of the solvent under atmospheric pressure. The extraction is preferably continued for a period of 10 minutes to 24 hours, though the extraction time varies depending on the extraction temperature.

To obtain an antibacterial fraction from the tea leaf extract thus obtained, the extract can be treated with a synthetic adsorbent. The synthetic adsorbent to be used in separating the tea leaf extract is, e.g., an aromatic compound-based synthetic adsorbent produced by polymerizing styrene and divinyl benzene or a methacrylic compound-based synthetic adsorbent produced by polymerizing methacrylic acid. Examples of commercially available aromatic compound-based synthetic adsorbents include DIAION HP20 and DIAION HP21 (manufactured by Mitsubishi Chemical Corporation), AMBERLITE XAD2 and AMBERLITE XAD 4 (manufactured by Rohm and Haas Company, USA) and so on, while examples of commercially available methacrylic compound-based synthetic adsorbents include DIAION HP1MG and DIAION HP2MG (manufactured by Mitsubishi Chemical Corporation), AMBERLITE XAD7 and AMBERLITE XAD 8 (manufactured by Rohm and Haas Company, USA) and so on.

It is preferable to carry out the synthetic adsorbent treatment by packing the synthetic adsorbent into a column, passing the tea leaf extract through the column and then washing the resin with water. When treating the tea leaf extract with the synthetic adsorbents, it is preferable to perform a pretreatment such as concentration under reduced pressure, to eliminate organic solvent from the extract, or sufficient dilution with water, to achieve complete fractionation of the extract. The gallocatechin gallate can be eluted from the adsorbent with the use of, for example, a 30% aqueous methanol solution, though the invention is not restricted thereto.

It is still preferable to further purify the eluate using DIAION HP-21 (manufactured by Mitsubishi Chemical Corporation) having a smaller pore size as a column packing. In this way, high-molecular weight substances such as polyphenols having molecular weight of 2000 or more, which were adsorbed together with the antibacterial components on an adsorbent in the case of HP-20, are not adsorbed and pass as such, and therefore, catechins and their epimers with lower molecular weights can be selectively adsorbed and collected. If necessary, the eluate is further treated with DIAION HP-21 or SEPHADEX LH-20 (manufactured by Amersham-Pharmacia) and eluted with a water-containing alcohol, e.g., a 30% aqueous methanol solution or water-containing acetone, e.g., a 50% aqueous acetone solution. In this way, substances not contributing to the antibacterial activity, e.g., caffeine, can be removed, and the catechins, EGCG and its epimer gallocatechin gallate, can be obtained at elevated concentration. The catechins, EGCG and its epimer gallocatechin gallate according to the present invention, can be determined by the colorimetry using the phenol reagent (Folin-Ciocalten reagent). However, it is advisable to use high-performance liquid chromatography (HPLC) to determine the composition in detail.

According to the above-described method, a composition is provided, wherein the antibacterial effect of EGCG is enhanced by GCG, and wherein the ratio EGCG:GCG ranges from 1.0:0.01 to 1.0:10.0, preferably from 1.0:0.05 to 1.0:2.0. From the fraction separated by the above-described method, a favorable GCG composition ratio can be determined using the antibacterial activity as an indication.

The tea leaf extract thus obtained may be used in any form, for example, a product as extracted or a product as eluted from the synthetic adsorbent, a concentrate thereof or a dried product obtained by removing solvents from the eluate. From the viewpoints of storage stability and safety for organic solvents, it is advisable to use the extract in the form of a dried product.

The composition according to the present invention may be used to reduce or prevent the onset of dental caries, by being added alone or together with various components already employed in the art, to drinks and foods of various purposes. To reduce or prevent the onset of dental caries means both to use the composition according to the present invention as oral care goods to suppress or prevent the onset of dental caries, and to regularly or intermittently take foods or drinks containing the composition according to the present invention over a certain period of time to reduce or prevent the onset of dental caries.

Preferred embodiments of the present invention include dental care goods such as toothpastes, mouth wash and troches, as well as additives to foods such as sweeteners such as sucrose, sweetened bean pastes, Castella cakes, *mizu-yokan* (soft adzuki-bean jelly), *dorayaki* (bean -jelly pancake) coating, sponge cakes, butter cakes, bavarois, custard cream, butter cream, custard pudding, cookies, sweet buns, steamed buns, jams, lactic acid bacteria drinks, carbonated drinks, coffee drinks, coffee jelly, caramel candies, ice creams, chewing gums, juice, candies and chocolates.

To produce these oral care goods or foods containing the cariostatic agents, components commonly employed in the art can be selected and used appropriately depending on the product types. For example, oral care goods may optionally contain calcium carbonate, dibasic calcium phosphate, silicic anhydride, magnesium carbonate, glycerine, sorbitol, propylene glycol, polyethylene glycol, carboxymethylcellulose, methyl cellulose, sodium alginate, carrageenan, crboxyvinyl polymer, sodium dioctylsulfosuccinate, sodium lauryl sulfate, sodium dodecylbenzenesulfonate, butyl parahydroxybenzoate, hinokitiol, allantoin, glycyrrhizin, alcohols, gum arabic, starch, corn starch, saccharin sodium, stevioside, glucose, lactose, magnesium stearate, monopotassium phosphate, dipotassium phosphate, menthol, eucalyptus oil, peppermint, spearmint, colorants, as well as fluorides such as sodium fluoride and sodium monofluorophosphate, anti-inflammatory agents such as lysozyme chloride and azulene, sodium chloride and so on.

On the other hand, foods may be produced by optionally blending commonly employed food materials such as glucose, fructose, sucrose, maltose, sorbitol, stevioside, corn syrup, lactose, citric acid, tartaric acid, malic acid, succinic acid, lactic acid, L-ascorbic acid, dl-α-tocopherol, sodium erythorbate, glycerine, propylene glycol, glycerine fatty acid esters, polyglycerine fatty acid esters, sucrose fatty acid esters, sorbitan fatty acid esters, propylene glycol fatty acid esters, gum arabic, carrageenan, casein, gelatin, pectin, agar, vitamin B family, nicotinic acid amide, calcium pantothenate, amino acids, calcium salts, colorants, flavoring agents and preservatives.

In the case of adding sugar together with a cariostatic agent to give such a cariostatic food, as will be shown in EXAMPLES by adding the composition according to the present invention to a food, a cariostatic sugar can be used as a substitute, as will be shown in EXAMPLES 6 to 8.

Since tea has been widely consumed all over the world from ancient times, there is no trouble with the fraction obtained from tea leaf extract in terms of safety. However, considering the flavor, smell, color tone, etc. of the product, it is preferred to add the composition according to the present invention (including the cariostatic composition) to the food and drink of the present invention at a concentration of 0.0001 to 0.5%, still preferably 0.01 to 0.2%, on a dry weight basis. It is also preferable to control the concentration in a food within the range as specified above, in use. Further, according to the present invention, it is also possible to reduce an amount of antibacterial catechins owing to the effect of gallocatechin gallate.

### ADVANTAGES OF THE INVENTION

The composition according to the present invention shows a strong antibacterial activity against Streptococcus mutans which is the major causative bacterium of dental plaque formation inducing dental caries and periodontal diseases. The component employed in the present invention is extremely safe and the composition can be supplied in a large amount as cariostatic and anti periodontal disease composition, because the ingredients of the composition originate in tea, which has been widely consumed from ancient times. Accordingly, the present invention appears to contribute to improvement in oral hygiene and, furthermore, is expected to be applicable to the field of general foods, and therefore, industrially very useful.

Next, the present invention will be illustrated in greater detail by reference to the following EXAMPLES. However, it is needless to say that the present invention is not restricted to these EXAMPLES.

### EXAMPLE 1: Enhancement of antibacterial effect by mixing EGCG and GCG

Streptococcus mutans (MT8148R) was cultured in a brain heart infusion medium (BHI, manufactured by Difco) at 37°C for 18 hours. After collection of the cells by centrifugation, the precipitate was washed three times with a phosphate buffer solution (PBS, pH 6.8). Then the cells were suspended to give an absorbance of 0.5 at 550 nm. Thus, about 2x10⁷ colonies per mL (expressed in colony forming unit: CFU) were formed . Next, an equal amount of a previously prepared sample solution was added to the suspension and the resultant mixture was allowed to stand at 37°C for 1 hour. 0.1 mL of this liquid mixture or the same mixture seriously 10-fold diluted was sowed on a Mitis salivarius agar medium (MS agar medium, manufactured by Difco). After culturing at 37°C for 2 days, the colonies thus formed were counted. The antibacterial activity was indicated in the logarithm of CFU of test group/CFU of control lot (i.e., the reducing ratio).

The mixing ratio of EGCG to GCG (manufactured by Sigma) was varied so as to adjust the final concentration of the sample to 1 mg/mL and thus the antibacterial activity was examined. Compared with the samples containing EGCG or GCG alone, the mixtures of them showed enhanced antibacterial activities. In particular, the sample having an EGCG:GCG ratio of 1.00:0.45 showed an activity 10^{1.23} times higher (about 15-fold) than that of the sample containing EGCG alone. Table 1 summarizes the results.

**Table 1**

| Ratio | | Antibacterial activity |
|---|---|---|
| EGCG | GCG | |
| - | 1.00 | -3.17 |
| 1.00 | - | -3.50 |
| 1.00 | 0.05 | -4.47 |
| 1.00 | 0.15 | -4.49 |
| 1.00 | 0.30 | -4.58 |
| 1.00 | 0.45 | -4.73 |
| 1.00 | 1.00 | -4.25 |
| 1.00 | 2.00 | -4.22 |

### EXAMPLE 2: Fractionation of oolong tea extract

To oolong tea leaves was added 10 volumes of a 45% (w/w) aqueous ethanol solution. After immersion of the tea leaves at room temperature for 1 day, the mixture was filtered and the extract thus obtained was powdered by concentration under reduced pressure and freeze-drying. To the powder thus obtained, 100 volumes of deionized water were added and the resultant suspension was passed through a DIAION HP-21 column (manufactured by Mitsubishi Chemical Corporation). After washing with deionized water, elution was carried out with the use of a 30% aqueous methanol solution (passing speed: SV=3, passing volume: 5-bed volumes each time). The eluate obtained using the 30% aqueous methanol solution was concentrated under reduced pressure and freeze-dried to give a powder.

Using the obtained powder, a 50% DMSO solution was prepared and analyzed by high-performance liquid chromatography (analyzer: Alliance Photodiode Array System, manufactured by Waters; column: Develosil C30-UG-5, 4.6x150 mm, manufactured by Nomura Kagaku Co., Ltd.; mobile phase and elution conditions: linear gradient from Solution A (0.05% trifluoroacetic acid) to Solution B (50% acetonitrile-0.05% trifluoroacetic acid) within 20 minutes; flow rate: 1 mL/min; column temperature: 40°C). C, CG, EC, GC, EGC, EGCG, GCG and ECG (manufactured by Sigma) were used as standards and the weights of the catechins contained were determined from the respective calibration curves. The composition ratio is shown in Table 2, with the major component EGCG being taken as 1.00.

**Table 2:**

| Composition ratio of catechins | | | | | | | |
|---|---|---|---|---|---|---|---|
| Composition ratio of catechins by weight | | | | | | | |
| EGCG | EGC | GCG | ECG | C | EC | GC | CG |
| 1.00 | 0.34 | 0.32 | 0.19 | 0.07 | Tr. | Tr. | Tr. |

In this Table, "Tr." means a trace amount which is too small to indicate numerically.

### EXAMPLE 3

### Toothpaste:

| (component) | (parts by weight) |
|---|---|
| Dibasic Calcium phosphate | 42.0 |
| Glycerine | 18.0 |
| Carrageenan | 0.9 |
| Sodium lauryl sulfate | 1.2 |
| Saccharin Sodium | 0.09 |
| Butyl parahydroxybenzoate | 0.005 |
| Tea leaf extract* | 0.2 |
| Flavoring agent | 1.0 |
| Water | the balance |
| Total | 100.0 |

| | |
|---|---|
| * The oolong tea leaf extract powder obtained in EXAMPLE 2 (the same will be applied hereinafter). | |

### EXAMPLE 4

### Mouth wash:

| (component) | (parts by weight) |
|---|---|
| Sodium lauryl sulfate | 0.8 |
| Glycerine | 7.0 |
| Sorbitol | 5.0 |
| Ethyl alcohol | 15.0 |
| Tea leaf extract | 0.2 |
| 1-Menthol | 0.05 |
| Flavoring agent | 0.04 |
| Saccharin sodium | 0.1 |
| Water | the balance |
| Total | 100.0 |

### EXAMPLE 5

### Troch:

| (component) | (parts by weight) |
|---|---|
| Gum arabic | 6.0 |
| Maltitol | 74.3 |
| Tea leaf extract | 0.2 |
| Sodium monofluorophosphate | 0.7 |
| Lactose | 18.3 |
| 1-Menthol | 0.5 |
| Total | 100.0 |

### EXAMPLE 6

### Production of cariostatic sugar (powder):

### (Production method)

A solution of the following composition was made by heating to 80 to 90°C, then transferred to a stainless squarish vat and dried in a drying apparatus at 105°C. During the drying, the solution was agitated at intervals of 1 hour. After the. completion of drying, it was ground in a mortar to give a powdery sugar.

| (component) | |
|---|---|
| Sugar | 200 parts |
| Tea leaf extract | 1 part |
| Water | 30 parts |

### EXAMPLE 7

### Production of cariostatic sugar (granules):

### (Production method)

A sugar according to the present invention was spray-granulated using a spray granulator (FLOW COATER MULTI TLO-5M, manufactured by Okawara Seisakusho Co. Ltd.) with the composition listed below. Namely, sugar was fed into a material container and pre-dried in a hot air stream at 90°C for about 2 hours. Next, a tea leaf extract dissolved in water was sprayed onto the sugar (100 ml/min, 30 seconds) with a spray-gun. After stopping the spraying, the mixture was intermediately dried for 20 minutes. After repeating the spraying and the intermediate drying 4 times, final drying was performed for 20 minutes followed by cooling for 20 minutes, thereby giving the cariostatic sugar.

| (Component) | |
|---|---|
| Sugar | 200 parts |
| Tea leaf extract | 1 part |
| Water | 10 parts |

### EXAMPLE 8

### Production of cariostatic sugar (syrup):

### (Production method)

To 50 parts of hot water was added 0.75 parts of the powdery oolong tea leaf extract obtained in EXAMPLE 2 and dissolved. Then 150 parts of sugar was added to the solution to give a cariostatic sugar (syrup) according to the present invention. It is preferable that the cariostatic sugars obtained in each of the above EXAMPLEs 6 to 8 contains 0.1 to 10 parts of the cariostatic agent per 1000 parts of sugar.

### EXAMPLE 9

### Chewing gum:

| (component) | (parts by weight) |
|---|---|
| Chewing gum base | 20.0 |
| Calcium carbonate | 2.0 |
| Stevioside | 0.1 |
| Tea leaf extract | 0.05 |
| Lactose | 76.85 |
| Flavoring agent | 1.0 |
| Total | 100.0 |

### EXAMPLE 10

### Juice:

| (component) | (parts by weight) |
|---|---|
| Frozen concentrated satsuma orange | 5.0 |
| Fructose/glucose liquid sugar | 11.0 |
| Citric acid | 0.2 |
| L-ascorbic acid | 0.02 |
| Tea leaf extract | 0.01 |
| Flavoring agent | 0.2 |
| Colorant | 0.1 |
| Water | the balance |
| Total | 100.0 |

### EXAMPLE 11

### Candy:

| (component) | (parts by weight) |
|---|---|
| Powdery sorbitol | 99.74 |
| Flavoring agent | 0.2 |
| Tea leaf extract | 0.01 |
| Sorbitol seed | 0.05 |
| Total | 100.0 |

### EXAMPLE 12

### Sweetened bean paste:

| (component) | (parts by weight) |
|---|---|
| Fresh red sweetened bean paste | 41.69 |
| Granulated sugar | 26.7 |
| Starch syrup | 7.8 |
| Tea leaf extract | 0.01 |
| Water | the balance |
| Total | 100.0 |

### EXAMPLE 13

### Castella cake:

| (component) | (parts by weight) |
|---|---|
| Soft flour | 17.38 |
| Whole egg | 39.7 |
| White superior soft sugar | 32.1 |
| Starch syrup | 6.7 |
| Tea leaf extract | 0.02 |
| Water | the balance |
| Total | 100.0 |

### EXAMPLE 14

### Mizuyokan:

| (component) | (parts by weight) |
|---|---|
| Fresh red sweetened bean paste | 24.8 |
| Powdery agar | 0.3 |
| Sodium chloride | 0.1 |
| White superior soft sugar | 24.9 |
| Tea leaf extract | 0.01 |
| Water | the balance |
| Total | 100.0 |

### EXAMPLE 15

### Dorayaki Coating:

| (component) | (parts by weight) |
|---|---|
| Soft flour | 33.0 |
| White superior soft sugar | 33.1 |
| Whole egg | 33.18 |
| Baking powder | 0.7 |
| Tea leaf extract | 0.02 |
| Total | 100.0 |

### EXAMPLE 16

### Sponge cake:

| (component) | (parts by weight) |
|---|---|
| Soft flour | 30.3 |
| White superior soft sugar | 39.38 |
| Whole egg | 30.3 |
| Tea leaf extract | 0.02 |
| Total | 100.0 |

### EXAMPLE 17

### Butter cake:

| (component) | (parts by weight) |
|---|---|
| Soft flour | 20.0 |
| Hard flour | 5.0 |
| Whole egg | 24.99 |
| Margarine | 25.0 |
| White superior soft sugar | 25.0 |
| Tea leaf extract | 0.01 |
| Total | 100.0 |

### EXAMPLE 18

### Bavarois:

| (component) | (parts by weight) |
|---|---|
| Cow's milk | 48.49 |
| Fresh cream | 16.2 |
| Yolk | 6.5 |
| Gelatin | 2.9 |
| White superior soft sugar | 12.9 |
| Tea leaf extract | 0.01 |
| Water | the balance |
| Total | 100.0 |

### EXAMPLE 19

### Custard cream:

| (component) | (parts by weight) |
|---|---|
| Cow's milk | 66.29 |
| Whole egg | 13.2 |
| Corn starch | 4.0 |
| White superior soft sugar | 16.5 |
| Tea leaf extract | 0.01 |
| Total | 100.0 |

### EXAMPLE 20

### Butter cream:

| (component) | (parts by weight) |
|---|---|
| Cow's milk | 21.7 |
| Margarine | 43.59 |
| Yolk | 13.0 |
| Granulated sugar | 21.7 |
| Tea leaf extract | 0.01 |
| Total | 100.0 |

### EXAMPLE 21

### Custard pudding:

| (component) | (parts by weight) |
|---|---|
| Cow's milk | 47.59 |
| Whole egg | 31.9 |
| White superior soft sugar | 17.1 |
| Tea leaf extract | 0.01 |
| Water | the balance |
| Total | 100.0 |

### EXAMPLE 22:

### Cookie:

| (component) | (parts by weight) |
|---|---|
| Soft flour | 31.887 |
| Whole egg | 16.0 |
| Margarine | 19.2 |
| White superior soft sugar | 25.5 |
| Baking powder | 0.2 |
| Tea leaf extract | 0.01 |
| Water | the balance |
| Total | 100.0 |

### EXAMPLE 23

### Sweet bun:

| (component) | (parts by weight) |
|---|---|
| Hard flour | 42.4 |
| Soft flour | 10.6 |
| White superior soft sugar | 10.6 |
| Isomerized sugar | 3.5 |
| Whole egg | 5.3 |
| Shortening | 3.2 |
| Dry yeast | 1.1 |
| Sodium chloride | 0.3 |
| Tea leaf extract | 0.01 |
| Water | the balance |
| Total | 100.0 |

### EXAMPLE 24

### Steamed bun:

| (component) | (parts by weight) |
|---|---|
| Soft flour | 26.2 |
| Whole egg | 32.78 |
| Granulated sugar | 26.2 |
| Butter | 6.6 |
| Salad oil | 5.9 |
| Baking powder | 2.3 |
| Tea leaf extract | 0.02 |
| Total | 100.0 |

### EXAMPLE 25

### Jam:

| (component) | (parts by weight) |
|---|---|
| Strawberry | 54.88 |
| Granulated sugar | 42.0 |
| Citric acid | 0.6 |
| Pectin | 2.4 |
| Flavoring agent | 0.1 |
| Tea leaf extract | 0.02 |
| Total | 100.0 |

### EXAMPLE 26

### Lactic acid bacteria drink:

| (component) | (parts by weight) |
|---|---|
| Fermented milk (solid milk components: 21%) | 14.76 |
| Fructose/glucose liquid sugar | 13.31 |
| Pectin | 0.5 |
| Citric acid | 0.08 |
| Flavoring agent | 0.15 |
| Tea leaf extract | 0.01 |
| Water | the balance |
| Total | 100.0 |

### EXAMPLE 27

### Carbonated drink:

| (component) | (parts by weight) |
|---|---|
| Granulated sugar | 8.0 |
| Concentrated lemon juice | 1.0 |
| L-Ascorbic acid | 0.10 |
| Citric acid | 0.06 |
| Sodium citrate | 0.05 |
| Colorant | 0.05 |
| Flavoring agent | 0.15 |
| Carbonated water | 90.58 |
| Tea leaf extract | 0.01 |
| Total | 100.0 |

### EXAMPLE 28

### Coffee drink:

| (component) | (parts by weight) |
|---|---|
| Granulated sugar | 8.0 |
| Skim milk powder | 5.0 |
| Caramel | 0.2 |
| Coffee extract | 2.0 |
| Flavoring agent | 0.1 |
| Polyglycerine fatty acid ester | 0.05 |
| Sodium chloride | 0.05 |
| Tea leaf extract | 0.01 |
| Water | the balance |
| Total | 100.0 |

### EXAMPLE 29

### Coffee jelly:

| (component) | (parts by weight) |
|---|---|
| Granulated sugar | 15.0 |
| Gelatin | 1.0 |
| Coffee extract | 5.0 |
| Tea leaf extract | 0.01 |
| Water | the balance |
| Total | 100.0 |

### EXAMPLE 30

### Caramel candy:

| (component) | (parts by weight) |
|---|---|
| Granulated sugar | 32.0 |
| Starch syrup | 20.0 |
| Milk powder | 40.0 |
| Hardened oil | 4.0 |
| Sodium chloride | 0.6 |
| Flavoring agent | 0.02 |
| Tea leaf extract | 0.02 |
| Water | the balance |
| Total | 100.0 |

### EXAMPLE 31

### Ice cream:

| (component) | (parts by weight) |
|---|---|
| Fresh cream (fat content:45%) | 33.8 |
| Skim milk powder | 11.0 |
| Granulated sugar | 14.8 |
| Sugar-containing yolk | 0.3 |
| Vanilla essence | 0.1 |
| Tea leaf extract | 0.01 |
| Water | the balance |
| Total | 100.0 |

### EXAMPLE 32

### Candy:

| (component) | (parts by weight) |
|---|---|
| Sugar | 47.0 |
| Starch syrup | 49.98 |
| Flavoring agent | 1.0 |
| Tea leaf extract | 0.02 |
| Water | the balance |
| Total | 100.0 |

### EXAMPLE 33

### Chocolate:

| (component) | (parts by weight) |
|---|---|
| Cacao mass | 18.0 |
| Cacao butter | 19.98 |
| Milk powder | 15.5 |
| Sugar | 46.0 |
| Lecithin | 0.5 |
| Tea leaf extract | 0.02 |
| Total | 100.0 |

## Claims

1. A composition comprising antibacterial catechins mixed with gallocatechin gallate.

2. A cariostatic composition comprising, as the main component, a synthetic adsorbent-adsorbed fraction which is obtained by a method comprising the steps of adsorbing a solvent-extract from a tea leaf on a synthetic adsorbent selected from among aromatic compound-based synthetic adsorbents and methacrylic compound-based synthetic adsorbents, and then eluting the adsorbed components from the adsorbent.

3. The cariostatic composition as claimed in claim 2 wherein the tea leaf is an oolong tea leaf.

4. The composition as claimed in any one of claims 1 to 3 wherein the composition ratio of gallocatechin gallate is determined using an antibacterial activity as an indication.

5. The composition as claimed in any one of claims 1 to 4 which contains epigallocatechin gallate and gallocatechin gallate in a ratio of 1:0.01 to 1:10.

6. A food or drink containing a composition as claimed in any one of claims 1 to 5, as an additive.

7. The food or drink as claimed in claim 6 wherein the content of the composition as claimed in any one of claims 1 to 5 is 0.0001% by weight to 0.5% by weight on a dry weight basis.

8. An oral care product for preventing dental caries which contains a composition as claimed in any one of claims 1 to 5.

9. The oral care product for preventing dental caries as claimed in claim 8 wherein the content of the cariostatic composition is 0.0001% by weight to 0.5% by weight on a dry weight basis.

10. A method which comprises mixing antibacterial catechins with gallocatechin gallate, whereby the antibacterial activity of the antibacterial catechins is enhanced compared with the activity before the mixing.

11. The method as claimed in claim 10 wherein the antibacterial activity of the antibacterial catechins is an activity against *Streptococcus mutans*.

12. The method as claimed in claim 10 or 11 wherein the antibacterial catechins consist of epigallocatechin gallate or mainly comprise epigallocatechin gallate.

13. The method as claimed in any one of claims 10 to 12 wherein the composition ratio of gallocatechin gallate is determined using the antibacterial activity as an indication.

14. The method as claimed in any one of claims 10 to 13 wherein the ratio of epigallocatechin gallate to gallocatechin gallate is 1:0.01 to 1:10.

15. Use of a composition as claimed in any one of claims 1 to 5 for producing a food or a drink.

16. Use of a composition as claimed in any one of claims 1 to 5 for producing an oral care product for preventing dental caries.
